# EUROPEAN PATENT APPLICATION

(11) **EP 3 756 619 A1**
(43) Date of publication of application: **30.12.2020**
(21) Application number: 20275109.5
(22) Date of filing: 12.06.2020
(51) Int. Cl.: A61F 2/07, A61F 2/82

(54) **TYPE II SAC ACCESS PORT IN AN ENDOGRAFT DEVICE**

(30) Priority: 26.06.2019 US 201916453476
(71) Applicant: Cook Medical Technologies LLC, Bloomington, IN 47404 (US)
(72) Inventor: MAJERCAK, David C., Bloomington, IN 47401 (US); SUMANASINGHE, Ruwan D., Carmel, IN 46032 (US); TUFFLEY, Ian, Bloomington, IN 47403 (US); SVENDSEN, Mark, Bloomington, IN 47401 (US)
(74) Representative: Williams Powell

(57) **Abstract**

An endograft device includes: an expandable tubular body having an inner side and an outer side of a surrounding wall, the tubular body encloses a lumen, having a first open end (116) and a second open end (118). A sheath pocket (120) having a pocket wall longitudinally disposed along the tubular body, enclosing a channel. The sheath pocket (120) having an input port (122) disposed proximal to the first open end (116), and an output port (126) disposed proximal to the second open end (118). The input port (122) faces towards the first open end (116) to provide an entrance to the sheath pocket (120). An expandable lining of wire framework (128), is inserted longitudinally into the lumen such that the lining of wire framework after expansion exerts an outward pressure against the inner side of the surrounding wall and against the pocket wall of the sheath pocket to naturally shut seal both the input port and the output port.

## Description

### TECHNICAL FIELD

The present disclosure relates to an endograft device for example for endovascular repair and an intervention method to treat conditions such as a Type II endoleak in Abdominal Aortic Aneurysm (AAA).

### BACKGROUND

Endovascular aneurysm repair (EVAR) of abdominal aortic aneurysms (AAA) since its introduction in 1991, has quickly gained acceptance as a minimally invasive alternative to open AAA repair (i.e., open surgery) to treat thoracic and abdominal aortic aneurysms and other aortic pathologies such as the acute aortic syndromes (e.g., penetrating aortic ulcer, intramural hematoma, dissection).

EVAR involves the placement of a prosthetic endograft device (such as a stent graft) within the thoracic or abdominal aorta at the site of an aneurysm. Endograft devices come in many different designs depending on their applications and the target site of deployment in the vascular system. Endograft devices are typically compressed within a delivery sheath and are introduced into the vascular system through the lumen of an access vessel to be subsequently deployed by a delivery tool at the site of the aneurysm. Once deployed at the target site of treatment, the endograft device self-expands to contact the aortic wall to protect a weakened aortic wall or to exclude and seal aneurysm sac in the formation of an aneurysm sac, which, if untreated may lead to aortic/aneurysm rupture due to increased blood flow or pressure build up at the untreated weakened wall. The endograft device therefore, should provide adequate seals or fixation both proximally and distally at the endograft device landing zones in order to exclude the aneurysm sac.

EVAR is currently the preferred mode of treatment of thoracic and abdominal aortic aneurysms. The advantages include a lower perioperative 30-day all-cause mortality as well as a significant reduction in perioperative morbidity when compared to open surgery, EVAR also leads to decreased blood loss, eliminates the need for cross-clamping the aorta and has shorter recovery periods than traditional surgery.

EVAR procedures nevertheless do have their challenges and disadvantages. The main disadvantage is post-procedural complications over a time-period after their deployment that often require secondary intervention. Common complications include both those related to the endograft device itself as well as systemic complications. Among the device-related complications, type II endoleak being the main one. Type II endoleak may be caused by persistent blood flow into and out the residual aneurysm sac due to a failure to completely exclude the aneurysm sac after the deployment of the endograft device at locations including aortic side branch vessels. Endoleak carries an increased risk for continued aneurysm expansion and eventual rupture if untreated.

Intervention is a procedure (herein after, including either an intra-operative intervention procedure during endograft device deployment or a re-intervention procedure after an endograft device has been previously deployed) that may address a type II endoleak including embolization or ligation of the aneurysm sac. Current designs of endograft devices once deployed are not very accessible to the aneurysm sac intervention procedure, which may be difficult to perform, time consuming and thus may experience a higher risk of failure.

### BRIEF SUMMARY

The present invention seeks to provide an improved endograft device and/or method.

According to an aspect of the invention, there is provided an endograft device as in claim 1.

The disclosure describes various embodiments of an endograft device with better access features to the aneurysm sac after deployment, and a method or a procedure of intervention using the described endograft device.

In some embodiments, the output port of the sheath pocket provides access to an aneurysm sac treat a type II endoleak.

In some embodiments, the type II endoleak in the aneurysm sac is treated by filling with a coagulant substance or an embolization coil.

In some embodiments, the coagulant substance is deposited on or mixed with the embolization coil.

In some embodiments, the embolization coil comprises polyethylene terephthalate (PET) fibers laced with the coagulant substance.

According to an aspect of the invention, there is provided an endograft device, comprising:
an tubular body having an inner-side surrounding wall and an outer-side surrounding wall, the tubular body encloses a lumen having a first open end and a second open end opposite to the first open end;
a sheath pocket having a pocket wall, forming an enclosed channel directly beneath the inner-side surrounding wall, the sheath pocket is longitudinally disposed along the tubular body, wherein the sheath pocket having an input port and an output port, wherein the input port faces towards the first open end to provide an entrance to the sheath pocket in the lumen, and the output port is an opening on the outer-side of the surrounding wall to provide the sheath pocket an external access to outside the tubular body; and
an expandable lining of wire framework, is inserted longitudinally into the lumen of the tubular body such that the lining of wire framework after expansion exerts an outward pressure from a lumen side against the inner-side of the surrounding wall and against the pocket wall of the sheath pocket to naturally shut seal both the input port and the output port.

The features of any of the dependent claims may be combined with this aspect. For conciseness they are not repeated here.

According to an aspect of the invention, there is provided an endograft device, comprising:
an tubular body having an inner-side surrounding wall and an outer-side surrounding wall, the tubular body encloses a lumen having a first open end and a second open end opposite to the first open end;
a sheath pocket having a pocket wall, forming an enclosed channel directly beneath the inner-side surrounding wall, the sheath pocket is longitudinally disposed along the tubular body, wherein the sheath pocket having an input port disposed proximal to the first open end, and an output port disposed proximal to the second open end, wherein the input port faces towards the first open end to provide an entrance to the sheath pocket in the lumen, and the output port is an opening on the outer-side of the surrounding wall to provide the sheath pocket an external access to outside the tubular body; and
an expandable lining of wire framework, is inserted longitudinally into the lumen of the tubular body such that the lining of wire framework after expansion exerts an outward pressure from a lumen side against the inner-side of the surrounding wall and against the pocket wall of the sheath pocket to naturally shut seal both the input port and the output port.

The features of any of the dependent claims may be combined with this aspect. For conciseness they are not repeated here.

According to an aspect of the invention, there is provided an intervention method to treat a type II endoleak in an aneurysm sac after deployment of the endograft device according to any of the claims, comprising:
accessing the aneurysm sac at a target site of a vascular system, wherein the accessing comprising:
   guiding a leading end of a delivery tool to locate through an identifier marker, the first open end of the endograft device which has previously been deployed at the target site of the vascular system, wherein the target side is a location of an aneurysm sac (endosac) where a type II endoleak has occurred;
   accessing, through locating a free-end of the pre-inserted tether wire, the input port of the sheath pocket; and
   guiding the leading end of the delivery tool through the sheath pocket, until the leading end exits the output port of the sheath pocket and into the aneurysm sac; and
delivering through the leading end of the delivery tool, a ligating substance into the aneurysm sac for coagulation.

The pre-inserted wire tether may be removed prior to the delivery of the ligating substance into the aneurysm sac.

The delivering of the ligating substance into the aneurysm sac may comprise delivering one of: an embolization coil having polyethylene terephthalate (PET) fibers laced with a coagulating agent and directly injecting coagulating agent.

The delivery tool may be a pre-loaded micro-catheter.

In one aspect of the disclosure, an endograft device includes: a tubular body having an inner-side surrounding wall and an outer-side surrounding wall, the tubular body encloses a lumen having a first open end and a second open end opposite to the first open end; a sheath pocket having a pocket wall, forming an enclosed channel directly beneath the inner-side surrounding wall, is longitudinally disposed along the tubular body, wherein the sheath pocket having an input port disposed proximal to, or close to, the first open end, and an output port disposed proximal to, or close to, the second open end, wherein the input port faces towards the first open end to provide an entrance to the sheath pocket from the lumen, and the output port is an opening on the outer-side of the surrounding wall to provide the sheath pocket an external access outside the tubular body, and an expandable lining of wire framework, is inserted longitudinally into the lumen of the tubular body such that the lining of wire framework after expansion exerts an outward pressure from a lumen side against the inner-side of the surrounding wall and against the pocket wall of the sheath pocket to naturally shut seal both the input port and the output port.

Another aspect of the disclosure presents an intervention method to treat a type II endoleak in an aneurysm sac after deployment of the endograft device as described. The method includes: accessing the aneurysm sac at a target side of a vascular system, guiding a leading end of a delivery tool to locate through an identifier marker, the first open end of the endograft device which has previously been deployed at the target site of the vascular system, accessing through locating a free-end of a pre-inserted tether wire, the input port of the sheath pocket; guiding the leading end of the delivery tool through the sheath pocket, until the leading end exits the output port of the sheath pocket and into the aneurysm sac; and delivering through the leading end of the delivery tool, a ligating substance into the aneurysm sac for coagulation.

The disclosure describes an endograft device that has a sheath pocket design and optionally include a pre-inserted tether wire. In embodiments, once deployed, a health practitioner has an option to perform in a sealed off environment, an intervention procedure using a delivery tool to quickly locate an entrance to the sheath pocket in the deployed endograft device through an identifier marker, and the physician may gain access to the aneurysm sac through the sheath pocket to fill the aneurysm sac with a coagulating substance. The endograft device thus enables performing an intervention procedure in either an acute or a chronic aneurysm with relatively less time and a lower skill requirement.

### BREIF DESCRIPTION OF THE DRAWINGS

Embodiments of the invention are described below, by way of example only, with reference to the accompanying drawings.
Fig. 1A illustrates an exemplary vascular system having deployed at a target site, a plurality of endograft devices to treat an aneurysm, according to a first embodiment.
Fig. 1B illustrates an exemplary vascular system having deployed at a target site, a plurality of endograft devices to treat an aneurysm, according to a second embodiment.
Fig. 2 depicts a side view of the first embodiment endograft device of Fig. 1A.
Fig. 3A depicts a sectional view 3-3, taken at an output port of the first embodiment endograft device of Fig. 2 according to a preferred embodiment.
Fig. 3B depicts a sectional view 3-3, taken at an output port of the first embodiment endograft device of Fig. 2 according to an alternate embodiment.
Fig. 4A depicts a sectional view 4-4, taken at an input port of the first embodiment endograft device of Fig. 2 according to a preferred embodiment.
Fig. 4B depicts a sectional view 4-4, taken at an input port of the first embodiment endograft device of Fig. 2 according to an alternate embodiment.
Fig. 5 depicts a top view 5-5, taken from above the first embodiment endograft device of Fig. 2.
Fig. 6 depicts a sectional view 6-6, taken from a lumen side of the first embodiment endograft device of Fig. 2.
Fig. 7A depicts the first embodiment endograft device of Fig. 2 with an option of pre-inserting a tether wire with a bow-tie shape loop-end at an output port of a sheath pocket.
Fig. 7B depicts the second embodiment endograft device of Fig. 1B with an option of pre-inserting a tether wire with a bow-tie shape loop-end under a flap structure at an output port of a sheath pocket.
Fig. 8A depicts a detailed top view at the output port of a sheath pocket, according to the first embodiment endograft device of Fig. 7A.
Fig. 8B depicts a detailed top view including the flap structure at the output port of a sheath pocket, according to the second embodiment endograft device of Fig. 7B.
Fig. 9A depicts a detailed cross section view at the output port of a sheath pocket, according to the preferred embodiment of the endograft device of Fig. 7A.
Fig. 9B depicts a detailed cross section view including the flap structure at the output port of a sheath pocket, according to the preferred embodiment of the endograft device of Fig. 7B.
Fig. 9C depicts a detailed cross section view including the flap structure at the output port of a sheath pocket, according to the alternate embodiment of the endograft device of Fig. 7A.
Fig. 9D depicts a detailed cross section view including the flap structure at the output port of a sheath pocket, according to the alternate embodiment of the endograft device of Fig. 7B.
Fig. 9E depicts a detailed cross section view of the pocket gap at the output port of a sheath pocket showing that the pocket gap is naturally shut sealed, according to the preferred embodiment of the endograft device of Fig. 7A.
Fig. 9F depicts a detailed cross section view of the pocket gap at the input port of a sheath pocket showing that the pocket gap is naturally shut sealed, according to the preferred integral wall embodiment of the endograft device of Fig. 7A.
Fig. 10A depicts a tether wire with a round-shape loop-end pre-inserted at an output port of a sheath pocket, according to the first embodiment endograft device of Fig. 2.
Fig. 10B depicts a tether wire with a round-shape loop-end pre-inserted under a flap structure at an output port of a sheath pocket, according to the second embodiment endograft device of Fig. 1B.
Fig. 11A depicts a detailed top view at the output port of a sheath pocket, according to the first embodiment endograft device of Fig. 10A.
Fig. 11B depicts a detailed top view including the flap structure at the output port of a sheath pocket, according to the second embodiment endograft device of Fig. 10B.
Fig. 12A depicts a detailed cross section view at the output port of a sheath pocket, according to the first embodiment endograft device of Fig. 10A in a preferred embodiment.
Fig. 12B depicts a detailed cross section view including the flap structure at the output port of a sheath pocket, according to the second embodiment endograft device of Fig. 10B in a preferred embodiment.
Fig. 12C depicts a detailed cross section view at the output port of a sheath pocket, according to the first embodiment endograft device of Fig. 10A in an alternate embodiment.
Fig. 12D depicts a detailed cross section view including the flap structure at the output port of a sheath pocket, according to the second embodiment endograft device of Fig. 10B in an alternate embodiment.
Fig. 13A is a perspective view of Fig. 7A, depicting the tether wire with a bow-tie shape loop-end pre-inserted into the sheath pocket, according to the first embodiment endograft.
Fig. 13B is a perspective view of Fig. 7B, depicting the tether wire with a bow-tie shape loop-end pre-inserted under the flap structure into the sheath pocket, according to the second embodiment endograft.
Fig. 14A depicts an embodiment of the tether wire with a bow-tie shape loop-end.
Fig. 14B depicts another embodiment of the tether wire with a round-shape loop-end.
Fig. 15 is a partial cut out view depicting a delivery tool performing an intervention procedure to treat type II endoleak in an aneurysm sac through the sheath pocket of a deployed first embodiment endograft in Fig. 1A.
Fig. 16 depicts the aneurysm sac having been filled with a coagulant substance after the intervention procedure in Fig. 15.
Fig. 17 is a sectional view 17-17 showing that the aneurysm sac having been filled with a coagulant substance and/or an embolization coil device after the intervention procedure as shown in Fig. 15.
Fig. 18 depicts a partially delivery tool which is used to advance through the sheath pocket of a deployed endograft device to access the aneurysm sac in order to deliver a coagulant substance into the aneurysm sac.

### DETAILED DESCRIPTION OF THE EMBODIMENTS

The various embodiments of the present disclosure are further described in details in combination with attached drawings and embodiments below. The specific embodiments described herein are used only to explain the present disclosure, and should not be construed as a limitation on the claims. In addition, for the sake of keeping description brief and concise, only the newly added features, or features that are different from those previously described in each new embodiment may be described in detail. Similar features may be referenced back to the prior descriptions in a prior numbered drawing or referenced ahead to a higher numbered drawing.

The target site illustrated may be at junctions between a renal artery 102 and iliac arteries 110, 112, which an Endovascular Aneurysm Repair (EAR) procedure may be performed to treat acute (sudden onset) or chronic (developed over an extended period of time) aneurysms. The plurality of endograft devices may include deployment of a main body endograft device 104 at the renal artery 102 section and two side-branch endograft devices, 106A, 108 at the iliac arteries sections.

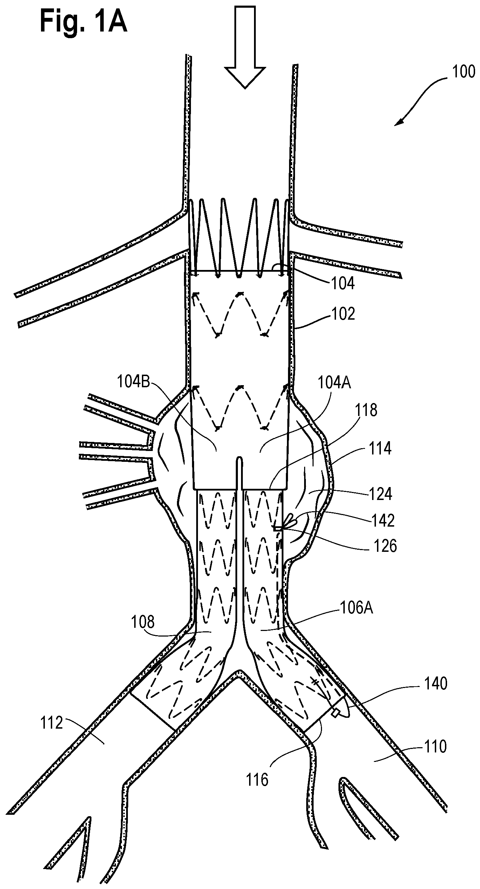

Fig. 1A illustrates an exemplary vascular system 100 having deployed at a target site, a plurality of endograft devices 104, 106A, 108 to treat an aneurysm, according to a first embodiment.

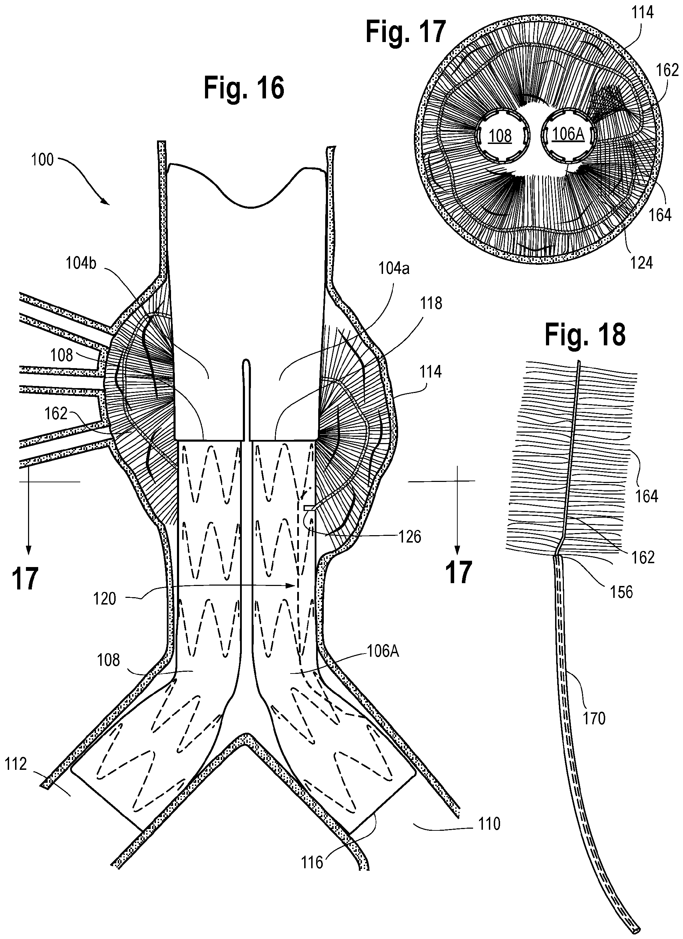

The exemplary illustrated EVAR procedure may involve the use of a delivery tool 170 (such as a catheter as shown in Fig.18) to deploy a plurality of the endograft devices one by one in a sequence. Each of the deployed endograft devices (including 104, 106A, 108) includes a stent (made from a lining of wire framework) which may self-expand to make contact with the aortic wall for a purpose of excluding an aneurysm sac 114, and to protect a weakened aortic wall due to occlusion or partial blockage of blood clot 124. Aneurysm, if without EVAR treatment may lead to aortic/aneurysm rupture caused by building up of blood pressure at the weakened aortic wall. The plurality of deployed endograft devices (104, 106A and 108) therefore, may need to provide adequate sealing as well as fixation in deployed position both proximally and distally at the endograft device landing zones in order to successfully exclude the aneurysm sac 114.

An exemplary deployment sequence may start with using a delivery tool 170 (such as an exemplary catheter shown in Fig. 18) to first deploy at the target site (such as a renal artery 102), the main body endograft device 104. The delivery tool 170 may mount a non-branching end of the main body endograft device 104 in a head first orientation, the main body endograft device 104 is then inserted head first into the renal artery 102 until reaching the target site, such that the two branching legs 104A, 104B at a tail end of the main body endograft device 104 may be pointing towards two corresponding branches of the iliac arteries 110, 112 after deployment.

The two respective side-branch endograft devices 106A, 108 may subsequently be deployed from the respective iliac arteries 110, 112 in order to connect to two corresponding legs 104A, 104B of the main body endograft device 104. In an embodiment, the delivery tool 170 may mount a second open end 118 (i.e., distal end) of the side-branch endograft device 106A in a head first orientation, the side-branch endograft device 106A is then inserted head first into the iliac artery 110, such that the second open end 118 may be inserted into and thus partially overlapped by a portion of the corresponding leg 104A of the main body endograft 104. A first open end 116 (i.e., proximal end) of the side-branch endograft device 106A may remain partly branched into the iliac artery 110. Likewise, another similar side-branch endograft device 108 may be deployed in like manner from the iliac artery 112 to connect to the leg 104B of the main body device 104, such that the connection junctions at the legs 104A, 104B may remain sealed off after deployment to result in unobstructed blood flow from the renal artery 102 into the iliac arteries 110, 112 without leakage of blood into the aneurysm sac 114.

In a case if the aneurysm sac 114 is not completely eliminated sometime (developed over an extended period of time) after deployment, a type II acute endoleak (sudden onset) or a type II chronic endoleak may be developed, in this example, from neighboring capillaries connected to the aneurysm sac 114 or at a junction between the leg 104A (or 104B) of the main body endograft device 104 and the side-branch endograft device 106A (or 106B), where blood may have slowly accumulate in the aneurysm sac 114. Intervention procedure may thus need to be performed in an attempt to coagulate the leaked blood in the aneurysm sac 114 to prevent further swelling which may cause rupture.

The first embodiment side-branch endograft device 106A may include an internal sheath pocket which forms an enclosed channel directly beneath an inner-side of the surrounding wall of the side-branch endograft device 106A, having an output port 126 with a slit opening or a slot opening as the output port 126 which may be exposed on an outer-side of the surrounding wall 132 of the side-branch endograft device 106A. In an embodiment, a tether wire 140 having a loop-end may be pre-inserted into the side-branch endograft device 106A to facilitate access to the aneurysm sac 114 for a an acute or chronic intervention procedure which would be further discussed.

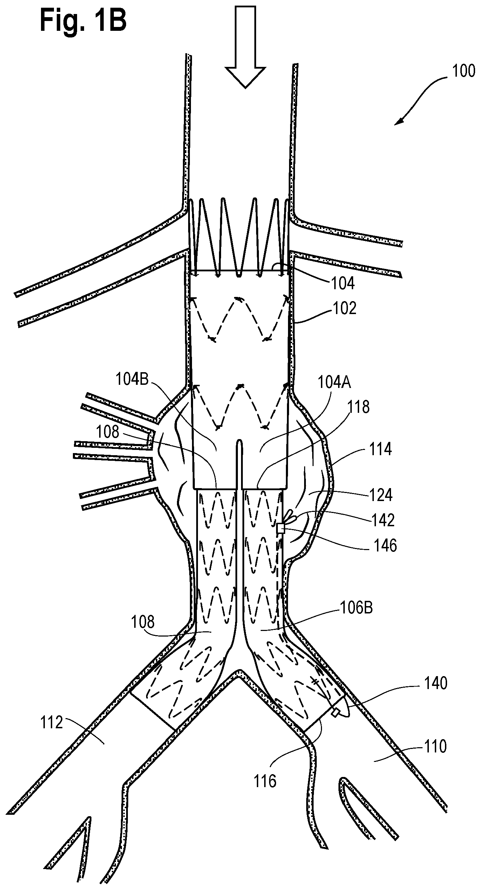

Fig. 1B illustrates an exemplary vascular system 100 having deployed at a target site, a plurality of endograft devices to treat an aneurysm, according to a second embodiment. Similarly, the second embodiment endograft device 106B may be deployed as a side-branch endograft device at junctions between the renal artery 102 and the iliac arteries 110, 112. In this second embodiment, a flap structure 146 may cover the output port 126 of the second embodiment endograft device 106B (compared to the first embodiment endograft device 106A which the output port 126 is exposed). The deployment procedure of the second embodiment endograft device 106B in the EVAR is no different than that of the first embodiment endograft device 106A.

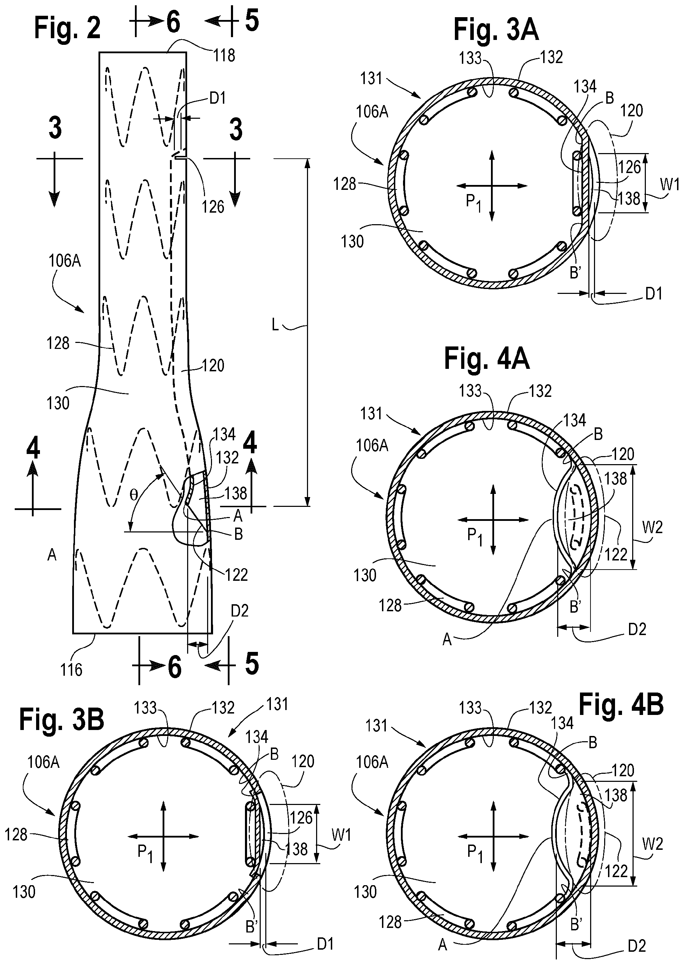

Fig. 2 depicts a side view of the first embodiment endograft device 106A of Fig. 1A. The pre-inserted tether wire 140 has been removed to simplify the features description of the sheath pocket 120. The first embodiment endograft device 106A may include a tubular body 131 having an inner-side 133 and an outer-side 132. The tubular body 131 encloses a lumen 130, having a first open end 116 and a second open end 118 opposite to the first open end 116. A sheath pocket 120, forming an enclosed channel (with a pocket gap 138) directly beneath the inner-side 133 of the tubular surrounding wall. The sheath pocket 120 is longitudinally disposed along the inner-side 133 of tubular surrounding wall, wherein the sheath pocket 120 having an input port 122 disposed proximal to (close to) the first open end 116, and an output port 126 disposed proximal to (close to) the second open end 118. The input port 122 faces towards the first open end 116 to provide an entrance to the sheath pocket 120 from the lumen, and the output port 126 is an opening on the outer-side 132 of the surrounding wall to provide the sheath pocket 120 an external access to outside the tubular body 131. An expandable lining of wire framework 128 (i.e., a stent) is inserted longitudinally into the lumen 130 of the tubular body 131 such that the lining of wire framework 128 after expansion, exerts an outward pressure P1 from a lumen side against the inner-side 133 of the tubular surrounding wall and against the sheath pocket 120 to naturally shut seal both the input port 122 and the output port 126.

In an embodiment, the material of the tubular body 131 may be a single piece of woven fabric of certain thickness made from clinically approved inert polyester polymers such as DACRON™, polytetrafluoroethylene (PTFE), or a suitable fabric coated with one or more clinically approved inert polyester coatings which are suitable for implant into a patient's vascular system.

The enclosed channel of the sheath pocket 120 may be formed by a pocket wall 134 enclosing a pocket gap 138 to allow insertion of a tether wire 140 or insertion of a delivery tool 170 to facilitate access to an aneurysm sac 114 (also known as endosac) in case of an intervention procedure by guiding the delivery tool 170 through the enclosed pocket gap 138 to exit the output port 126 outside the surrounding wall 132, such that a coagulating substance may be delivered into the aneurysm sac 114 to treat an acute or chronic type II endoleak.

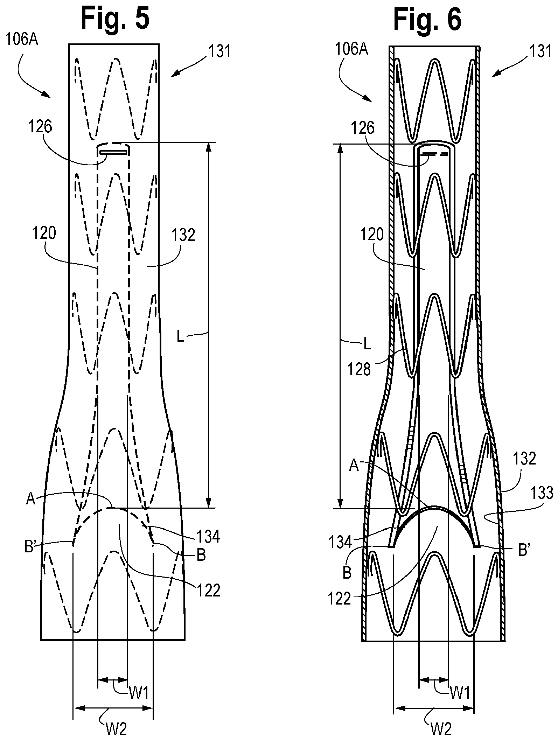

As mentioned above, the lining of wire framework 128 may exert an outward pressure P1 from the lumen side against the inner-side 133 of the surrounding wall and against a pocket wall 134 of the sheath pocket 120 to naturally shut seal both the input port 122 and the output port 126. Fig. 2 depicts that the sheath pocket 120 is fully stretched opened towards the lumen side to show the pocket gap 138, and an access diameter (or depth) D1 of the input port 122 is greater than an access diameter (or depth) D2 of the output port 126, and the input port 122 forms an angled inlet (an angle of θ degree, between 10° to 30°) with an inclined pocket wall 134 towards the lumen 130 that terminates at an apex A when viewed sideway in a cross section as shown in Fig. 2. Thus, the angled inlet formed by the inclined pocket wall 134 maximizes an access diameter D1 of the input port 122 when fully stretched open, while minimizes a pocket wall length L (also see Figs. 5-6) on the lumen side. The output port opening 126 may be one of: a slit opening or a slot opening that opens to the outer-side 132 of the surrounding wall.

In a preferred embodiment, the angled inlet of the input port 122 may remain stretched open at all times after deployment through structural reinforcement such as held open by a lining of wiring (e.g., stent wiring or nitinol), such that the angled inlet of the input port 122 may enable insertion guidance or easy access by the delivery tool 170 for performing an acute or a chronic endoleak intervention procedure. In another preferred embodiment, the access diameter D1 of the input port 122 and/or the access diameter D2 of output port 126 is at least 5 millimeter (5mm) which may be sufficiently large enough to accommodate any tool (such as the delivery tool 170) for performing an acute or a chronic endoleak intervention procedure.

Fig. 3A depicts a sectional view 3-3, taken at an output port 126 of a deployed first embodiment endograft device 108A of Fig. 2, according to a preferred embodiment. In the preferred embodiment in Fig. 3A (also see Figs. 4A, 9A, 9B, 12A, 12B), it is shown that the pocket wall 134 that encloses the sheath pocket 120 may be an integral part of the inner-side 133 of the surrounding wall itself. That is, both sides of a base B, B' (also see Fig. 5, 6) of the pocket wall 134 may be seamlessly merged with the inner-side 133 of the surrounding wall. In the preferred embodiment shown, the entire tubular body 131 may be made from a continuous single piece of woven fabric during a weaving process. In other embodiments, the tubular body 131 may be manufactured by a 3-dimensional (3D) printing process using suitable inert polymers or composite materials that is clinically proven to be safe for implanting in a vascular system.

Fig. 3A also shows that the lining of wire framework 128 in the lumen 130 may exert an outward expansion force forming a pressure on a surface which "pushes against" the inner-side 133 of surrounding wall and against the pocket wall 134. Fig. 3A also shows that the pocket gap 138 at the output port 126 when stretched opened, may have an access diameter or depth of D1 and a width of W1. In actuality, it should be noted that the pocket gap 138 throughout an entire longitudinal length L of the sheath pocket 120 and the output port 126 would become "flattened" or naturally sealed shut or closed by virtue of a sum pressure P1 from the lumen side 130, including a pressure caused by the outward expansion force from the lining of wire framework 128 against a surface of the pocket wall 134 (and the inner-side 133 of the surrounding wall) and an internal blood pressure of a patient (i.e., pulse blood pressure) in the lumen 130. In effect, the sum pressure P1 is naturally aided by the internal blood pressure from the lumen side to naturally shut seal both the input port and the output port, such that an increase in the internal blood pressure translates to a tighter seal to the pocket wall 134 of the sheath pocket 120.

Fig. 3B depicts a sectional view 3-3, taken at an output port 126 of a deployed first embodiment endograft device 108A of Fig. 2, according to an alternate embodiment. In the alternate embodiment in Fig. 3B (also see Figs. 4B, 9C, 9D, 12C, 12D), it is shown that the pocket wall 134 that encloses the sheath pocket 120 at the base B, B' that the pocket wall 134 that encloses the sheath pocket 120 may be a separate piece of woven fabric fixedly bonded to or sewn to the inner-side 133 of the surrounding wall. The features and functions of the alternate embodiment are similar to the preferred embodiment and they will not be repeated in the description. It is also to be noted that although embodiments are referred to herein as preferred and alternate, features of preferred and alternate embodiments may be combined and interchanged.

Fig. 4A depicts a sectional view 4-4, taken at an input port 122 of a deployed first embodiment endograft device 108A of Fig. 2, according to a preferred embodiment. In the preferred embodiment in Fig. 4A, it is shown that the pocket wall 134 that encloses the sheath pocket 120 may be an integral part of the inner-side 133 of the surrounding wall itself. That is, both sides of a base B, B' (also see Fig. 5, 6) of the pocket wall 134 may be seamlessly merged with the inner-side 133 of the surrounding wall. Also, in the preferred embodiment, the lining of wire framework 128 in the lumen 130 may exert an outward expansion force forming a pressure on a surface which "pushes against" the inner-side 133 of surrounding wall and against the pocket wall 134. Fig. 4A also shows that the pocket gap 138 at the input port 122 when stretched opened, may have an access diameter or depth of D2 and a width of W2, where D2 and W2 at the input port 122 is greater than D1 and W1 at the output port 126 thus forming a funnel shaped sheath pocket 120 (when stretched open) to enable ease of guiding a delivery tool 170 (as shown in Fig. 18) into the input port 122 and to advance through the pocket gap 138 to the output port 126 in an intervention procedure. Similar to Fig. 3A, it should be noted that the pocket gap 138 throughout an entire longitudinal length L of the sheath pocket 120 and the input port 122 would become "flattened" or naturally sealed closed by virtue of a sum pressure P1 from the lumen side 130, caused by both the outward expansion force exerted against the pocket wall 134 (and the inner-side 133 of the surrounding wall) from the lining of wire framework 128 and by the internal blood pressure in the lumen 130.

Fig. 4B depicts a sectional view 4-4, taken at an input port of the first embodiment endograft device of Fig. 2 according to an alternate embodiment. In the alternate embodiment in Fig. 4B, it is shown at the base B, B' that the pocket wall 134 that encloses the sheath pocket 120 may be a separate piece of woven fabric fixedly bonded to or sewn to the inner-side 133 of the surrounding wall. The features and functions of the alternate embodiment are similar to the preferred embodiment and they will not be repeated in the description.

Fig. 5 depicts a top view 5-5, taken from above the first embodiment endograft device 108A of Fig. 2, showing a pocket gap profile of the sheath pocket 120 disposed longitudinally along the inner-side of the surrounding wall (shown as dotted lines). The output port opening 126 may be one of: a slit opening or a slot opening that opens to the outer-side 132 of the surrounding wall.

The output port 126 may be a slot or through hole opening to the outer-side 132 of the surrounding wall with a width W1, and the input port 122 may be shown to include an angled inlet formed by an inclined pocket wall 134 at an angle towards the lumen 130. The angled inlet of the input port 122 may start from a base B and on the lumen 130 side).

Fig. 6 depicts a sectional view 6-6 (i.e., a projection view), taken from a lumen 130 side of the first embodiment endograft device 108A of Fig. 2. It is shown that the input port 122 that forms an angled inlet (an angle of θ degree, between 10° to 30° when viewed sideway in a cross section as shown in Fig. 2) includes an inclined pocket wall 134 which begins from a base B (or B') on one side at the inner-side 133 of the surrounding wall rises at an angle θ towards the lumen 130 to an apex A (i.e., maximum height), and returns to a base B' (or B) on another side at the inner-side 133 of the surrounding wall.

It should be noted that the angled inlet formed by the inclined pocket wall 134 in Fig. 6 when stretched opened to view from a projection angle, may appear to form a "D" shape or funnel shape" at the input port 122 of the sheath pocket 120. The input port 122 having the inclined angled inlet may have an effect of maximizing an access diameter D2 of the input port 122, while minimizing a length L on the pocket wall 134 on the lumen side. The "D shape or funnel shape" input port 122 alone (or in combination with an option of a pre-inserted tether wire) facilitates guiding of the delivery tool 170 to advance into the sheath pocket 120 with minimal obstructions during the intervention procedure, thus achieving the benefits of enabling a health practitioner to perform in a sealed off environment, an intervention procedure using a delivery tool 170 to easily locate the angled inlet input port 122 as an entrance, and to advance the delivery tool 170 into the sheath pocket 120 to quickly gain access to the aneurysm sac 114 to fill the aneurysm sac 114 with a coagulating substance. The endograft device 106A in various embodiments thus enables a health practitioner performing an intervention procedure in either an acute or a chronic aneurysm with relatively less time and a lower skill requirement.

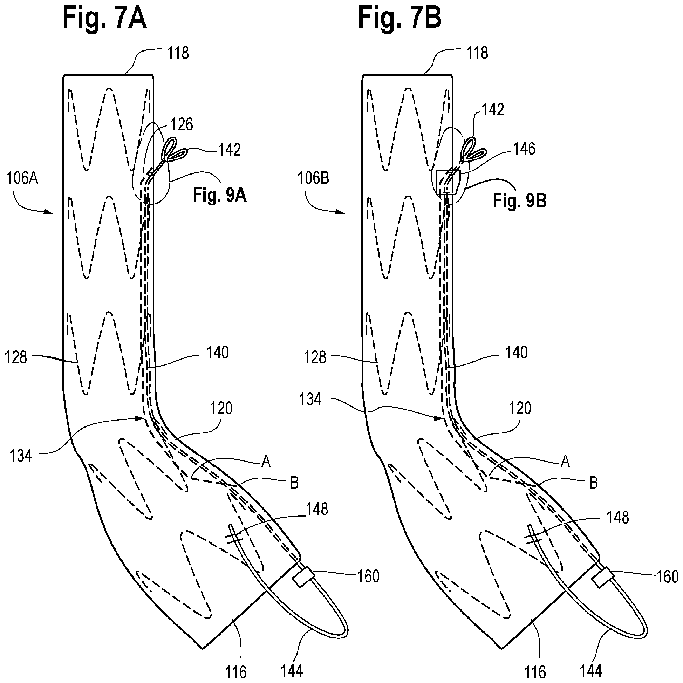

Fig. 7A depicts the first embodiment endograft device of Fig. 1A with an option of pre-inserting a tether wire 144 into the sheath pocket 120 at the output port 126. In an exemplary embodiment, the tether wire 144 may have a loop-end 142 shaped as a bow-tie. The loop-end 142 may come with other shapes, which may be inserted at an output port 126 of a sheath pocket 120, according to the first embodiment endograft device of Fig. 2. As mentioned in the description of Figs. 3A-3B and 4A-4B, the sheath pocket 120 is naturally "flattened" or sealed shut (to prevent endoleak) by an outward "pushing" force or pressure P1 exerted by both the expansion force of the lining of wire framework 128 and the internal blood pressure within the lumen 130. To facilitate ease of locating the input port 122 and to advance a delivery tool 170 through the sheath pocket 120, it may be desirable to pre-insert a tether wire 140 to help the delivery tool 170 to locate the input port 122 and open the pocket gap 138 of the sheath pocket 120 when ready to perform an intervention procedure.

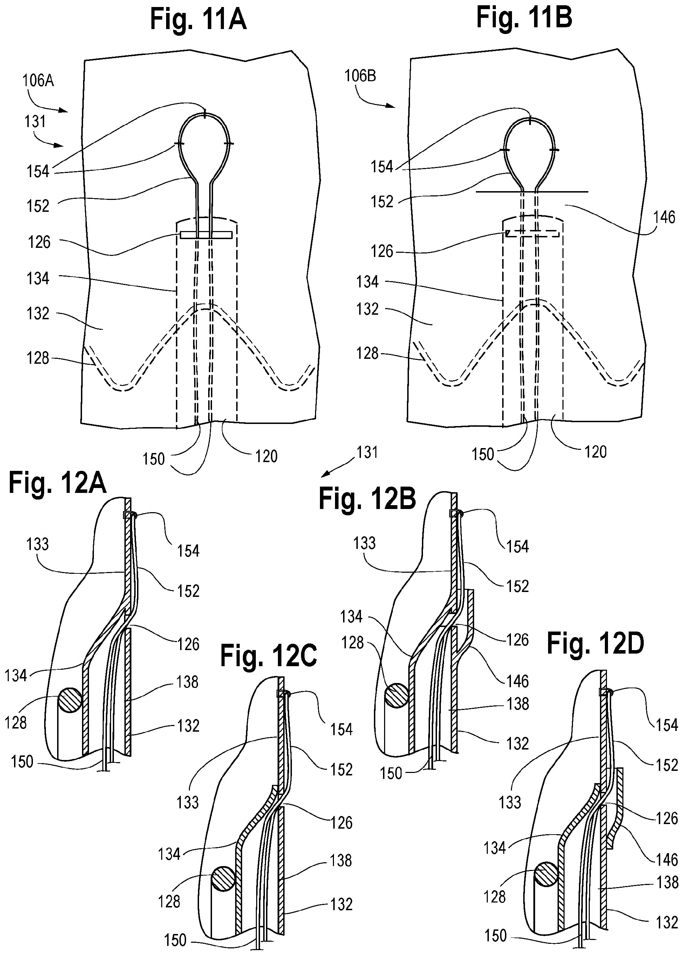

In an embodiment, the tether wire 140 may be a single wire (such as a nitinol wire) having a bow-tie shape loop-end 142 and a free-end 144. The free-end 144 of the tether wire 140 may be inserted at the output port 126, and pulled out at the input port 122 until the bow-tie shape loop-end 142 is sufficiently close to the output port 126 without obstructing sealing of the output port 126, while sufficient tether wire 140 length may be provided inside the sheath pocket 120 and at free-end 144 for ease of insertion. The bow-tie shape loop-end 142 and the free-end 144 of the tether wire 140 may provide sufficient stress relief to be fixedly and externally sutured 148 (such as stitching) onto the outer-side 132 of the surrounding wall. It should be noted that the entire tether wire 140 (including the bow-tie shape loop-end 142 or the round-shape loop-end 152 later described in Figs. 11A and 11B) may be removed by a predetermined force limit using a retraction tool after an acute or a chronic endoleak intervention procedure.

An identifier marker 160 (an inert metallic tag) may be crimped onto the free-end 144, and the identifier marker 160 may act as a radiopaque marker to locate the input port 122 and act as a capture target for retraction. It should be pointed out that the output port 126 of the sheath pocket 120 may be deliberately disposed with sufficient distance away from the second open end 118 to maximize overlapping and sealing of the junction between the leg 104A or 104B of the main body and the endograft devices 106A, 106B, while preserving strategic space or allowing clearance to access the aneurysm sac 114 in an intervention procedure.

Fig. 7B depicts the second embodiment endograft device 106B of Fig. 1B with an option of pre-inserting the tether wire 140 with a bow-tie shape loop-end 142 under a flap structure 146 at an output port 126 of a sheath pocket 120. More specifically, Fig. 7B discloses an option of using a flap structure 146 to cover the output port 126 to provide additional sealing of the sheath pocket 120 at the output port 126. The remaining features of the endograft device 106B are identical to the endograft device 106A and will not be repeated in this description.

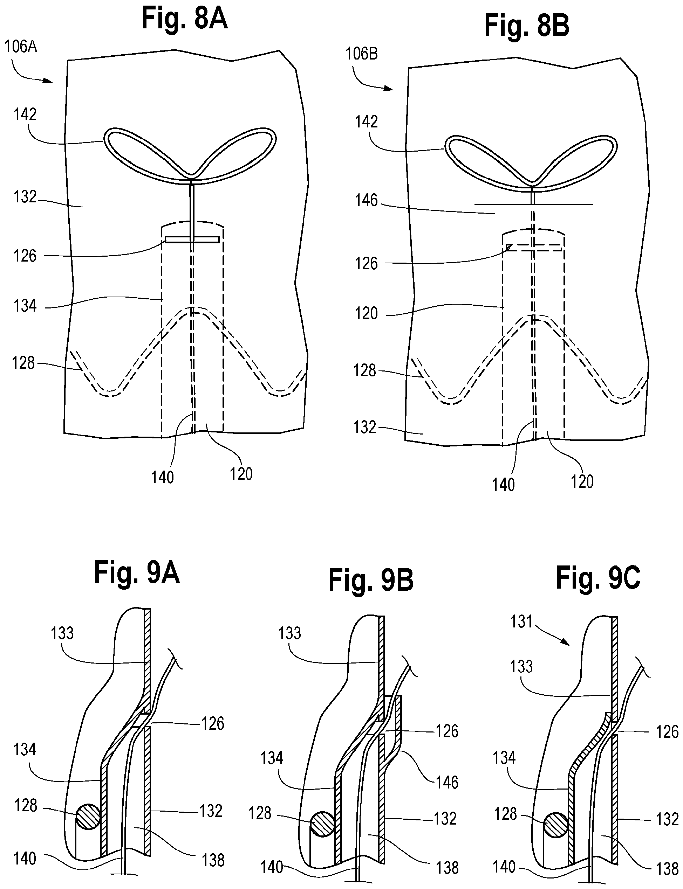

Fig. 8A depicts a detailed top view at the output port 126 of a sheath pocket 120), according to the first embodiment endograft device 106A of Fig. 7A. It is shown that the output port 126 may be a slit or a slot opening through the outer-side 132 of the surrounding wall. It should be noted that no suturing is necessary for the loop-end if the shape is a bow-tie shape 142.

Fig. 8B depicts a detailed top view including the flap structure 146 at the output port 126 of a sheath pocket 120, according to the second embodiment endograft device 106B of Fig. 7B. It may be shown that the flap structure 146 fully covers or overlaps the output port 126 while providing sufficient clearance to allow the insertion of the tether wire 140 into the sheath pocket 120. Similar to the description of Figs. 3A-3B, 4A-4B, the flap structure 146 (if present) may be an integrally and seamlessly merged with the outer-side 132 of the surrounding wall in the preferred embodiment, or the flap structure 146 may be a separate structure bonded onto or sewn onto the outer-side 132 of the surrounding wall in the alternate embodiment to provide additional sealing to the output port 126 and the sheath pocket 120.

Fig. 9A depicts a detailed cross section view at the output port 126 of a sheath pocket 120, according to the preferred embodiment of the endograft device 106A of Fig. 7A. It is shown that the pocket wall 134 in the lumen 130 may be seamlessly merged or integrally woven (or 3D printed) as a continuous layer onto the inner-side 133 of the surrounding wall to enclose the pocket gap 138, which the tether wire 140 may be inserted through the output port 126.

Fig. 9B depicts a detailed cross section view including the flap structure 146 at the output port 126 of a sheath pocket 120, according to the preferred embodiment of the endograft device 106B of Fig. 7B. It is shown that the flap structure 146 may be seamlessly merged or integrally woven (or 3D printed) as a continuous layer onto the outer-side 132 of the surrounding wall as a part of the tubular body 131 to fully cover the output port 126, while leaving sufficient clearance to allow the tether wire 140 to be inserted or to be retracted. The covering of the flap structure 146 may provide additional sealing to the output port 126 and the sheath pocket 120.

Fig. 9C depicts a detailed cross section view at the output port 126 of a sheath pocket 120, according to the alternate embodiment of the endograft device 106A of Fig. 7A. It is shown that the pocket wall 134 in the lumen 130 may be a separate piece of woven fabric bonded onto or sewn onto the inner-side 133 of the surrounding wall to enclose the pocket gap 138, which the tether wire 140 may be inserted through the output port 126.

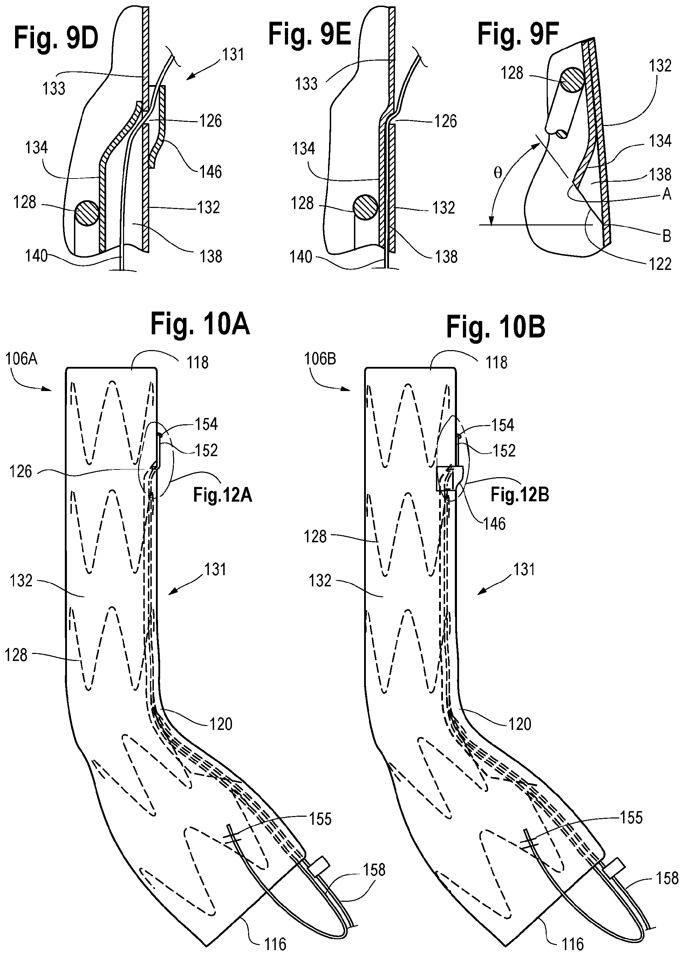

Fig. 9D depicts a detailed cross section view including the flap structure 146 at the output port 126 of the sheath pocket 120, according to the alternate embodiment of the endograft device 106B of Fig. 7B. It is shown that the flap structure 146 may be a separate piece of woven fabric bonded onto or sewn onto the outer-side 132 of the surrounding wall as an addition to the tubular body 131 to fully cover the output port 126, while leaving sufficient clearance to allow the tether wire 140 to be inserted or to be retracted.

Fig. 9E depicts a detailed cross section view of the pocket gap 138 at the output port 126 of the sheath pocket 120 showing that the pocket gap 138 is naturally shut sealed, according to the preferred embodiment of the endograft device 106A of Fig. 7A.

Fig. 9F depicts a detailed cross section view of the pocket gap near the input port 122 of a sheath pocket showing that the pocket gap before the angled inlet is naturally shut sealed, according to the preferred integral wall embodiment of the endograft device 106A of Fig. 7A.

In actuality, the lining of wire framework 128 (stent wire) from the lumen side 130 is shown to push against the pocket wall 134 and the inner-side 133 of the surrounding wall. As depicted in Figs. 9E to 9F, the pocket gap 138 may be shown as "flattened" or sealed shut from the output port 126 up to right before the angled inlet of the input port 122, due to the sum pressure P1 caused by the outward force of the lining of wire framework 128 on the pocket wall surface and caused by the internal blood pressure from the lumen as described in Figs. 3-4.

Fig. 10A depicts another embodiment of a tether wire 150 with a round-shape loop-end 152 pre-inserted at an output port 126 of a sheath pocket, according to the first embodiment endograft device106A of Fig. 2. It is shown that a round-shape loop-end 152 may be simply formed by looping the single tether wire 150 in the middle, and inserting both free-ends 158 of the tether wire 150 into the pocket gap 138 of the sheath pocket 120 through the output port 126. The round-shape loop-end 152 may be sutured 154 (stitched) at the loop-end onto the tubular body 131 (i.e., outer-side of the surrounding wall 132). Optionally, both free-ends 158 of the tether wire 150 may also be sutured 155 onto the tubular body 131 (i.e., outer-side of the surrounding wall 132) near the first open end 116.

Fig. 10B depicts a tether wire 150 with a round-shape loop-end 152 pre-inserted under a flap structure 146 at an output port 126 of a sheath pocket, according to the second embodiment endograft device 106B of Fig. 1B. More specifically, Fig. 10B discloses a flap structure 146 to provide additional sealing by covering the output port 126. The remaining features of the endograft device 106B are identical to the endograft device 106A and will not be repeated in the description.

Fig. 11A depicts a detailed top view at the output port 126 of a sheath pocket, according to the first embodiment endograft device of Fig. 10A. It is shown that a round-shape loop-end 152 may be formed by looping the single tether wire 150 in the middle, and inserting both free-ends of the tether wire 150 into the sheath pocket 120 through the output port 126. The round-shape loop-end 152 may be sutured 154 (stitched) at the loop-end on the external of the tubular main body (i.e., surrounding wall 132). It should be noted that the stitches of the sutures 154 for the round-shape loop-end 152 may be removed at the time of retraction after an acute or a chronic endoleak intervention procedure. In other embodiments, the retraction may simply involve retracting the tether wire 150 along the sutures 154 without removal of the sutures 154.

Fig. 11B depicts a detailed top view including the flap structure 146 at the output port 126 of a sheath pocket 120, according to the second embodiment endograft device of Fig. 10B. More specifically, Fig. 11B discloses a flap structure 146 to provide additional sealing by covering the output port 126 while the suturing 154 is outside the flap structure 146. The remaining features of the endograft device 106B are identical to the endograft device 106A and will not be repeated.

Fig. 12A depicts a detailed cross section view at the output port 126 of a sheath pocket 120, according to the preferred first embodiment endograft device 106A of Fig. 10A. Fig. 12A is similar to Fig. 8A, except to show the tether wire 150 having a round-shape loop-end 152 formed by looping the single tether wire 150 in the middle, and inserting both free-ends of the tether wire 150 into the sheath pocket 120 through the output port 126. The round-shape loop-end 152 may be sutured 154 (stitched) at the loop-end on the tubular body (i.e., outer-side 132 of the surrounding wall).

Fig. 12B depicts a detailed cross section view including the flap structure 146 at the output port 126 of a sheath pocket 120, according to the preferred second embodiment endograft device 106B of Fig. 10B. It is shown that the flap structure 146 fully covers or overlaps the output port 126 while providing sufficient clearance for the insertion of the round-shape loop-end 152 of the tether wire 150.

Fig. 12C depicts a detailed cross section view at the output port 126 of a sheath pocket 120, according to the alternate first embodiment endograft device 106A of Fig. 10A. In Fig. 12C, it is shown that the pocket wall 134 is a separate woven fabric bonded onto or sewn on the inner-side 133 of the surrounding wall.

Fig. 12D depicts a detailed cross section view including the flap structure 146 at the output port 126 of a sheath pocket 120, according to the alternate second embodiment endograft device 106B of Fig. 10B. It is shown that the flap structure 146 and the pocket wall 134 are separate woven fabric bonded onto or sewn on the outer-side 132 and the inner-side 133 of the surrounding wall, respectively.

It should be noted that as depicted in Figs. 12A to 12D, the lining of wire framework (stent wire) from the lumen side 130 is shown to push against the pocket wall 134 and the inner-side 133 of the surrounding wall. In actuality, the pocket gap 138 should be shown as "flattened" or sealed shut as shown and described in Figs. 9E and 9F.

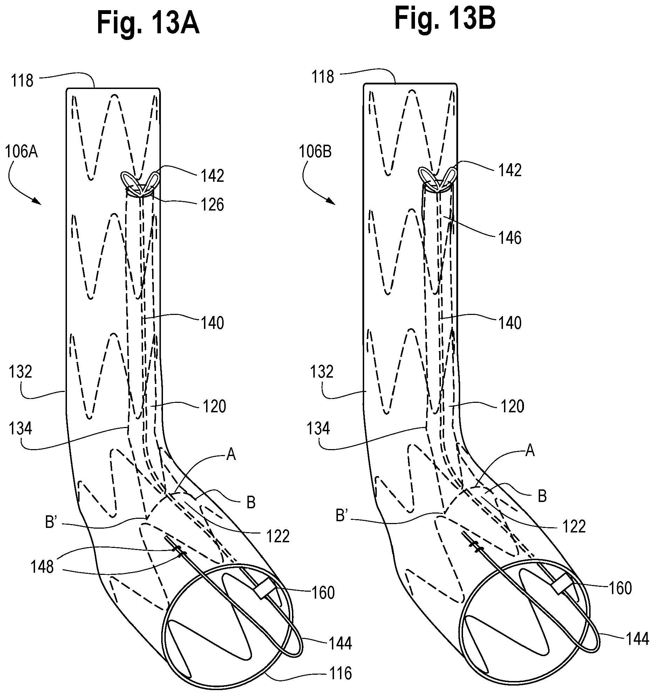

Fig. 13A is a perspective view of Fig. 7A, depicting the tether wire 144 with a bow-tie shape loop-end 142 pre-inserted into the sheath pocket 120, according to the preferred first embodiment endograft 106A. It is shown that the tether wire 140 may be a single wire (such as a nitinol wire) having a bow-tie shape loop-end 142 and a free-end 144. The free-end 144 of the tether wire 140 may be inserted at the output port 126, and pulled out at the input port 122 until the bow-tie shape loop-end 142 is sufficiently close to the output port 126, while providing sufficient tether coil length inside the sheath pocket 120 at the input port 122 for complete retraction after an intervention procedure. The free-end 144 of the tether wire 140 may provide sufficient stress relief to be externally sutured 148 (such as stitching) onto the surrounding wall 132.

An identifier marker 160 (an inert metallic tag) may be crimped on to the free-end 144 before the suturing 148 location, and the identifier marker 160 may act as a radiopaque marker to locate the input port 122 and act as a capture target for retraction. It should be pointed out that the output port 126 of the sheath pocket 120 is deliberately disposed with sufficient distance away from the second open end 118 to maximize sealing by being overlapped by the leg 104A or 104B of the main body endograft device 104, while preserving a strategic location with clearance to access the aneurysm sac 114 in an intervention procedure.

It should be noted that stents, springs or rings, or a combination of all may form the lining of wire framework 128 along the length of the sheath pocket 120 to realize the pressure exerted on the surrounding wall 132 of the endograft device 106A (or 106B).

Fig. 13B is a perspective view of Fig. 7B, depicting the tether wire 144 with a bow-tie shape loop-end 142 pre-inserted under the flap structure 146 into the sheath pocket 120, according to the second embodiment endograft 106B. The flap structure 146 provides additional sealing by covering the output port 126.

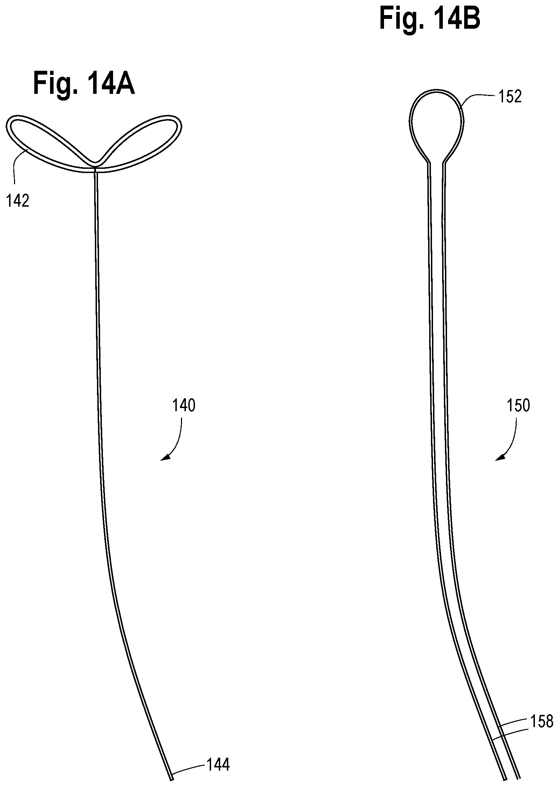

Fig. 14A depicts an embodiment of the tether wire 140 with a bow-tie shape loop-end 142 and a free-end 144. Fig. 14B depicts another embodiment of the tether wire with a round-shape loop-end 152. More specifically, one of the tether wire 140 and tether wire 150 may be pre-inserted into the sheath pocket 120, and the bow-tie shape loop-end (142) is not sutured to the exterior side of the outer surrounding wall 132, while the round-shape loop-end 152 may be fixedly sutured 154 to the tubular body 131 or the outer-side 132 of the outer surrounding wall. The pre-inserted tether wire 140 or 150 may be configured to be removed by snaring using a retraction tool only after an intervention procedure.

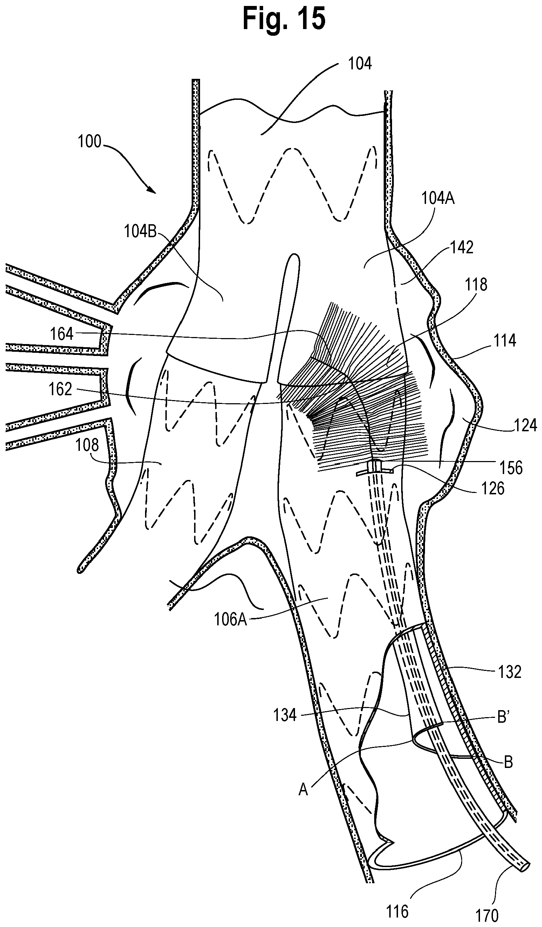

Fig. 15 is a partial cut out view depicting a delivery tool 170 (which may be a pre-loaded micro-catheter) performing an intervention procedure to treat type II endoleak in an aneurysm sac 114 through the sheath pocket 120, according to a deployed first embodiment endograft 106A in Fig. 1A. More specifically, the procedure including carrying out the following steps on a deployed endograft device 106A or 106B: Identifying a target site location of an aneurysm sac 114 where a type II endoleak has occurred in a vascular system. Accessing the aneurysm sac 114 at a target site of a vascular system, wherein the accessing includes: guiding a leading end of a delivery tool 170 to locate through an identifier marker 160, the first open end 116 of the endograft device 106A which has previously been deployed at the target site of the vascular system, wherein the target side is a location of an aneurysm sac 114 where a type II endoleak has occurred. Accessing, through locating a free end 144 of the pre-inserted tether wire 140, the input port 116 of the sheath pocket 120; and guiding the leading end of the delivery tool 170 through the sheath pocket 120, until the leading end exits the output port 126 of the sheath pocket and into the aneurysm sac 114; and delivering through the leading end 156 of the delivery tool 170, a ligating substance into the aneurysm sac 114 for coagulation.

Coagulation is also known as clotting, a process which blood may change from a liquid state to a gel, forming a blood clot. The mechanism may involve activation, adhesion and aggregation of platelets along with deposition and maturation of fibrin. Some examples of coagulant substance (antifibrinolytic drugs) are aprotinin, tranexamic acid (TXA), epsilon-aminocaproic acid and aminomethylbenzoic acid. Fig. 15 also shows that a fibrous embolization coil 162 having been mixed with a coagulant substance (antifibrinolytic drugs) may be injected through the delivery tool 170 into the aneurysm sac 114 during the intervention procedure. In other embodiments, the coagulant substance (antifibrinolytic drugs) may also be injected directly through the delivery tool 170 into the aneurysm sac 114 during the intervention procedure. The method of coagulant delivery during the intervention procedure is not limiting.

The partially cut out view of Fig. 15 also illustrates the sheath pocket 120 enclosed by the receding pocket wall 134, which the angled inlet ("recessed D shape") input port 122 may be formed by an inclined pocket wall 134 protruding from a base B (or B') toward the lumen 130 which rises to an apex A and return to the other side of the base B' (or B) on the inner-side 133 of the surrounding wall. The inclined or angled pocket wall 134 of the input port 122 acts like a funnel to facilitate or to guide the delivery tool 170 to open and to advance the tool 170 into the sheath pocket 120 with minimal obstructions during the intervention procedure.

It may be noted that the tether wire 140 may be retracted prior to the delivery of the coagulant. The procedure of retraction of the tether wire may vary and will not be discussed in details here.

Fig. 16 depicts the aneurysm sac 114 having been filled with a coagulant substance after the intervention procedure in Fig. 15. Fig. 16 also shows that the tether wire 140 may have been retracted or removed prior to the delivery of the ligating substance into the aneurysm sac or after the intervention procedure.

Fig. 17 is a sectional view 17-17 showing that the aneurysm sac 114 having been filled with a coagulant substance and/or an embolization coil with antifibrinolytic drugs after the intervention procedure as shown in Fig. 15.

Fig. 18 depicts a partially delivery tool 170 which is used to advance through the sheath pocket 120 of a deployed endograft device 106A or 106B to access the aneurysm sac 114 in order to deliver a coagulant substance into the aneurysm sac 114. As shown in Fig. 18, a fibrous (such as polyethylene terephthalate (PET) fibers) embolization coil 162 mixed with a coagulant substance (antifibrinolytic drugs) may be delivered by the delivery tool 170 at the output port 126 of the sheath pocket 120.

The disclosure describes an endograft 106A, 106B that has a sheath pocket design and a pre-inserted tether. Once deployed, a physician has the option to perform in a sealed off environment, an intervention procedure using a delivery tool to quickly locate an entrance to the sheath pocket in the deployed endograft through an identifier marker, and gain access to the aneurysm sac through the sheath pocket to fill the aneurysm sac with a coagulating substance.

It should be apparent to those skilled in the art that various modifications on sheath pocket 120, the input port 122 and output port 126 designs may be made to the present disclosure without departing from the scope or spirit of the disclosure. In view of the foregoing, the present disclosure covers modifications and variations which fall within the scope of the following claims and their equivalents.

It will be apparent to those skilled in the art that various modifications and variations can be made to the present disclosure without departing from the scope or spirit of the disclosure. In view of the foregoing, it is intended that the present disclosure cover modifications and variations of this disclosure provided may fall within the scope of the following claims and their equivalents.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application number 16/453,476, from which this application claims priority, and in the abstract accompanying this application, are incorporated herein by reference.

**Legends**

| | |
|---|---|
| 100 | vascular system |
| 102 | renal artery |
| 104 | main body endograft device |
| 104A, 104B | legs |
| 106A, B, | (side-branch) endograft device |
| 108 | (side-branch) endograft device |
| 110, 112 | Iliac artery |
| 114 | aneurysm sac |
| 116 | first open end |
| 118 | second open end |
| 120 | sheath pocket |
| 122 | input port |
| 124 | blood clot |
| 126 | output port |
| 128 | lining of wire framework |
| 130 | lumen |
| 131 | tubular body |
| 132 | outer-side of the surrounding wall |
| 133 | inner-side of surrounding wall |
| 134 | pocket wall |
| 138 | pocket gap |
| 140 | tether wire |
| 142 | bow-tie shape loop-end |
| 144, 158 | free-end (of tether wire) |
| 146 | flap structure |
| 148 | suture |
| 150 | round shape loop-end |
| 154, 155 | suture |
| 156 | leading end (of a delivery tool) |
| 160 | identifier marker (radiopaque) |
| 162 | embolization coil |
| 164 | PET fibers |
| 170 | delivery tool (catheter) |
| L | pocket length |
| A | apex |
| B, B' | base |
| W1. W2 | width |
| D1, D2 | access diameter or depth |
| P1 | sum pressure (an expansion force from the lining of wire frame on a surface + internal blood pressure) |

## Claims

1. An endograft device, comprising:
an tubular body having an inner-side surrounding wall and an outer-side surrounding wall, the tubular body encloses a lumen having a first open end and a second open end opposite to the first open end;
a sheath pocket having a pocket wall, forming an enclosed channel directly beneath the inner-side surrounding wall, the sheath pocket is longitudinally disposed along the tubular body, wherein the sheath pocket having an input port disposed close to the first open end, and an output port disposed close to the second open end, wherein the input port faces towards the first open end to provide an entrance to the sheath pocket in the lumen, and the output port is an opening on the outer-side of the surrounding wall to provide the sheath pocket an external access to outside the tubular body; and
an expandable lining of wire framework, is inserted longitudinally into the lumen of the tubular body such that the lining of wire framework after expansion exerts an outward pressure from a lumen side against the inner-side of the surrounding wall and against the pocket wall of the sheath pocket to naturally shut seal both the input port and the output port.

2. The endograft device according to claim 1, wherein the output port opening comprises one of: a slit opening or a slot opening that opens to the outer-side of the surrounding wall.

3. The endograft device according to claim 2, wherein the slit opening or the slot opening is entirely covered by an external flap structure which is one of: an integral part of the outer-side of the surrounding wall or a separate part fixedly bonded to or sewn to the outer-side of the surrounding wall.

4. The endograft device according to any preceding claim, wherein a width of the input port is wider than a width of the output port, such that when the sheath pocket is fully stretched opened towards the lumen side: wherein an access diameter of the input port is greater than an access diameter of the output port, and the input port forms an angled inlet with an inclined pocket wall towards the lumen that terminates at an apex when viewed sideway in a cross section.

5. The endograft device according to claim 4, wherein the angled inlet of the input port remains stretched open to enable insertion guidance or easy access by a tool for performing an acute or a chronic endoleak intervention procedure.

6. The endograft device according to claim 4 or 5, wherein the access diameter of the input port and the output port is at least 5 mm wide to accommodate a tool for performing an acute or a chronic endoleak intervention procedure.

7. The endograft device according to any preceding claim, further comprising a pre-inserted wire tether which facilitates locating and accessing of the input port and the output port of the sheath pocket, wherein the pre-inserted wire having a loop-end which extends beyond the output port, and a free-end which extends beyond the input port of the sheath pocket, wherein the free-end of the wire tether is sutured to the outer-side of the surrounding wall of the tubular body.

8. The endograft device according to claim 7, wherein the loop-end keeps the pre-inserted tether wire from being pulled into the sheath pocket up to a predetermined force limit, and the loop-end having a shape comprises one of: a bow-tie shape and a round loop.

9. The endograft device according to claim 8, wherein the bow-tie shaped loop-end is not sutured to the outer-side of the surrounding wall, while the round-shape loop-end is fixedly sutured to the outer-side of the surrounding wall of the tubular body.

10. The endograft device according to any of claims 7 to 9, wherein the pre-inserted wire tether is configured to be removed after performing an acute or a chronic endoleak intervention procedure.

11. The endograft device according to any of claims 7 to 10, wherein the pre-inserted wire tether is a single nitinol wire.

12. The endograft device according to any of claims 7 to 11, wherein an identifier marker is fixedly attached to the free-end portion of the pre-inserted wire tether to aid locating of the input port.

13. The endograft device according to claim 12, wherein the identifier marker comprises at least one radiopaque or metal tag.

14. The endograft device according to any preceding claim, wherein the output port of the sheath pocket provides access to an aneurysm sac treat a type II endoleak; optionally wherein the type II endoleak in the aneurysm sac is treated by filling with a coagulant substance or an embolization coil; optionally wherein the coagulant substance is deposited on or mixed with the embolization coil; optionally wherein the embolization coil comprises polyethylene terephthalate (PET) fibers laced with the coagulant substance.

15. The endograft device according to any preceding claim, wherein the outward pressure is aided by an internal blood pressure from the lumen side against the inner-side of the surrounding wall and against the pocket wall of the sheath pocket to naturally shut seal both the input port and the output port, such that an increase in the internal blood pressure causes a tighter seal to the pocket wall of the sheath pocket.
